# EUROPEAN PATENT SPECIFICATION

(11) **EP 1 984 026 B1**
(45) Date of publication and mention of the grant of the patent: **01.05.2013**
(21) Application number: 07717141.1
(22) Date of filing: 30.01.2007
(51) Int. Cl.: A61K 45/06, A61K 31/22, A61K 31/366, A61K 31/40, A61K 31/404, A61K 31/505, A61K 31/401, A61P 9/10

(54) **COMPOSITIONS TREATING CARDIOVASCULAR, CEREBROVASCULAR AND OTHER VASCULAR DISEASE PATIENTS**
ZUSAMMENSETZUNGEN ZUR BEHANDLUNG VON PATIENTEN MIT KARDIOVASKULÄREN, CEREBROVASKULÄREN UND ANDEREN GEFÄSSERKRANKUNGEN
COMPOSITIONS POUR TRAITER DES PATIENTS ATTEINTS DE MALADIES CARDIOVASCULAIRES, CÉRÉBROVASCULAIRES ET AUTRES MALADIES VASCULAIRES

(30) Priority: 30.01.2006 US 763308 P
(43) Date of publication of application: 29.10.2008
(73) Proprietor: Bryson Israel, Ben David, Sandersville, GA 31082 (US)
(72) Inventor: Bryson Israel, Ben David, Sandersville, GA 31082 (US)
(74) Representative: Boff, James Charles
(86) International application number: PCT/US2007/002550
(87) International publication number: WO 2007/089787

(56) References cited:
- ATHYROS V G ET AL: "ATORVASTATIN PLUS PRAVASTATIN FOR THE TREATMENT OF HETEROZYGOUS FAMILIAL HYPERCHOLESTEROLAEMIA - A PILOT STUDY" CURRENT MEDICAL RESEARCH AND OPINION, HANTS, GB, vol. 17, no. 4, 2001, pages 267-272, XP009026572
- VAN DAM MARJEL J ET AL: "A comparison of the efficacy and tolerability of titrate-to-goal regimens of simvastatin and fluvastatin: A randomized, double-blind study in adult patients at moderate to high risk for cardiovascular disease" CLINICAL THERAPEUTICS, vol. 23, no. 3, March 2001 (2001-03), pages 467-478, XP009090535 ISSN: 0149-2918
- LEITER L A ET AL: "Efficacy and safety of cerivastatin in primary hypercholesterolemia: A long term comparative titration study with simvastatin" CANADIAN JOURNAL OF CARDIOLOGY, vol. 15, no. 5, May 1999 (1999-05), pages 545-555, XP009090551 ISSN: 0828-282X
- BALLANTYNE CHRISTIE M ET AL: "Efficacy and safety of ezetimibe co-administered with simvastatin compared with atorvastatin in adults with hypercholesterolemia" AMERICAN JOURNAL OF CARDIOLOGY, vol. 93, no. 12, 15 June 2004 (2004-06-15), pages 1487-1494, XP009090544 ISSN: 0002-9149
- SCHEEN A J: "[The IDEAL study comparing simvastatin 20-40 mg versus atorvastatin 80 mg for secondary prevention after myocardial infarction: between two ideas of the ideal]" REVUE MÉDICALE DE LIÈGE JAN 2006, vol. 61, no. 1, January 2006 (2006-01), pages 53-59, XP009090552 ISSN: 0370-629X
- MCKENNEY JAMES M ET AL: "Use of a treatment algorithm to achieve NCEP ATP III goals with atorvastatin" JOURNAL OF CARDIOVASCULAR PHARMACOLOGY, vol. 46, no. 5, November 2005 (2005-11), pages 594-599, XP009090590 ISSN: 0160-2446
- FOLEY KATHLEEN A ET AL: "Effectiveness of statin titration on low-density lipoprotein cholesterol goal attainment in patients at high risk of atherogenic events." AMERICAN JOURNAL OF CARDIOLOGY, vol. 92, no. 1, 1 July 2003 (2003-07-01), pages 79-81, XP009090539 ISSN: 0002-9149
- BAYS HAROLD E ET AL: "Time as a variable with niacin extended-release/lovastatin vs. atorvastatin and simvastatin." PREVENTIVE CARDIOLOGY FALL 2005, vol. 8, no. 4, October 2005 (2005-10), pages 226-233, XP009090553 ISSN: 1520-037X
- STEIN E ET AL: "Achieving lipoprotein goals in patients at high risk with severe hypercholesterolemia: Efficacy and safety of ezetimibe co-administered with atorvastatin" AMERICAN HEART JOURNAL, MOSBY- YEAR BOOK INC., ST. LOUIS, MO, US, vol. 148, no. 3, September 2004 (2004-09), pages 447-455, XP004573143 ISSN: 0002-8703
- BERNE CHRISTIAN ET AL: "Comparison of rosuvastatin and atorvastatin for lipid lowering in patients with type 2 diabetes mellitus: results from the URANUS study" CARDIOVASCULAR DIABETOLOGY, BIOMED CENTRAL, LONDON, GB, vol. 4, no. 7, 3 June 2005 (2005-06-03), pages 1-11, XP021007166 ISSN: 1475-2840
- SCHUSTER H: "Improving lipid management - To titrate, combine or switch" INTERNATIONAL JOURNAL OF CLINICAL PRACTICE 2004 UNITED KINGDOM, vol. 58, no. 7, 2004, pages 689-694, XP002455522 ISSN: 1368-5031

## Description

This invention claims priority under 35 USC §119(e) to US Provisional Application 60/763,308, filed January 30, 2006 .

### Field of the Invention

The present invention is directed to a composition comprising one or more statins from two different groups, 1) one or more lipophilic statins and 2) one or more hydrophilic statins. The present invention is also directed towards compositions for the treatment of patients with cardiovascular diseases and other vascular diseases, including but not limited to Diabetes Mellitus type I and/or II (DM), coronary artery diseases (CAD), peripheral vascular diseases (PVD), cerebrovascular disease, post myocardial infarctions (post MI), post cerebrovascular accidents (post CVA), Hypertriglyceridemia, Hypercholesterolemia, and abdominal aortic aneurysms (AAA).

### Background of the invention

Low density lipoproteins (LDL) carry cholesterol around in the blood. The measurement of LDL-cholesterol is known to be an indicator of one's risk of heart attack and stroke. The lower one's LDL cholesterol, the lower one's risk. Generally, the level of LDL cholesterol is a better gauge of risk than total blood cholesterol. Table 1 shows generalized LDL cholesterol level ranges and goals for healthy individuals:

| LDL Cholesterol Levels | Relative Health Level |
|---|---|
| Less than 100 mg/dL | Optimal |
| 100 to 129 mg/dL | Near Optimal/ Above Optimal |
| 130 to 159 mg/dL | Borderline High |
| 160 to 189 mg/dL | High |
| 190 mg/dL and above | Very High |

However, the latest guidelines issued by NCEP (National Cholesterol Education Program) for high risk patients is to have a LDL less than 70 mg/dL. These high risk patients tend to have one or more other risk factors such as, for example, high blood pressure, they smoke, they are obese, diabetes, low HDL, high plaque volumes, or other factors. It should be understood that when cholesterol levels are referenced herein and the units are not explicitly stated, the units are mg/dL.

Statins are medicines that lower blood cholesterol levels by inhibiting HMG-CoA reductase (3-hydroxy-3-methylglutaryl coenzyme A reductase), an enzyme that is involved in the biosynthesis of cholesterol. Generally, statins are any of a number of drugs (for example, lovastatins and simvastatins) that inhibit the synthesis of cholesterol and promote the production of LDL-binding receptors in the liver resulting in a decrease in the level of LDL and a modest increase in the level of HDL (high density lipoproteins, also known as "good cholesterol") circulating in blood plasma.

The use of a single statin at recommended starting doses tends to reduce one's LDL-cholesterol somewhat but usually not too significantly (20-40% average response). Doubling the dose of a statin leads to only another 6% to 10% decrease in LDL levels.

Statins that are available for use by the public include the lipophilic statins atorvastatin (LIPITOR™), simvastatin (ZOCOR™), lovastatin (MEVACOR™), and the hydrophilic statins pravastatin (PRAVACHOL™), fluvastatin (LESCOL™) and rosuvastatin (CRESTOR™). There are also combination therapies available such as ezetimibe plus simvastatin (VYTORIN™) and niacin plus lovastatin (ADVICOR™).

The current standard of therapy is to continue to increase one statin dose to the maximal recommended dose in order to further reduce LDL cholesterol levels. However, this increase in dosage has led to LDL cholesterol decreases that are small (6 to maybe 10% maximal). Moreover, when taking higher doses of the statins, the side effect rates easily double (varies per statin and patient).

Athyros et al, Atorvastatin Plus Pravastatin for the Treatment of Heterozygous Familial Hypercholesterolaemia - A Pilot Study discloses administering a combination of atorvastatin and pravastatin to patients suffering from coronary artery disease who presented with amino transferase levels greater than three times the normal limit. The dosage administered was 20mg/day of atorvastatin plus 40mg/day of pravastatin and did not cause hepatotoxicity in patients who were sensitive to higher doses of statins in monotherapy.

### Brief summary of the invention

The present invention is directed to a composition comprising one or more statins from two different groups, 1) one or more lipophilic statins and 2) one or more hydrophilic statins. The present invention is also directed towards the preparation of cholesterol lowering compositions for treating patients with cardiovascular diseases and other vascular related diseases, including but not limited to Diabetes Mellitus type I and/or II (DM), coronary artery diseases (CAD), peripheral vascular diseases (PVD), post myocardial infarctions (post MI), post cerebrovascular accidents (post CVA), Hypertriglyceridemia, Hypercholesterolemia, and abdominal aortic aneurysms (AAA) using the composition of the present invention.

### Detailed description of the invention

The present invention relates to a composition comprising one or more statins from two different groups, 1) one or more lipophilic statins and 2) one or more hydrophilic statins. The present invention also relates to the preparation of cholesterol lowering compositions for treating patients with cardiovascular diseases and other vascular related diseases, including but not limited to Diabetes Mellitus type I and/or II (DM), coronary artery diseases (CAD), peripheral vascular diseases (PVD), post myocardial infarctions (post MI), post cerebrovascular accidents (post CVA), Hypertriglyceridemia, Hypercholesterolemia, and abdominal aortic aneurysms (AAA).

To date, to the inventor's knowledge, no one has made a composition comprising one or more statins that fall into a lipophilic category such as atorvastatin (LIPITOR™), simvastatin (ZOCOR™), and lovastatin (MEVACOR™), with one or more statins that fall into a hydrophilic category such as pravastatin (PRAVACHOL™), fluvastatin (LESCOL™) and rosuvastatin (CRESTOR™). Although, these statins are (or were) approved for use by the FDA in the United States, it should be understood that the present invention encompasses statins that are not currently approved for use. The compositions of a lipophilic statin and a hydrophilic statin that are modifications of the above statin compounds are contemplated and therefore within the scope of the present invention.

The modifications of the above enumerated statins include modifications wherein a methyl or methylene group can be modified to a higher order alkyl or alkylene group, respectively. For example, a methyl group can be changed to an ethyl group, a n-propyl group, an i-propyl group, a n-butyl group, an i-butyl group, or a t-butyl group. Likewise, a methylene linker can be changed to an ethylene linker, a propylene linker or a butylene linker (wherein any or all of the hydrogens in the linker group can be replaced with a methyl group). Moreover, hydrogens can be modified to methyl groups. Modifications also include the substitution of an acidic proton with any pharmaceutically acceptable salt. These pharmaceutically acceptable salts can be found in, for example, Remington: The Science and Practice of Pharmacy, 20^{th} edition, ed. Alfonso R. Gennaro, Baltimore, MD: Lippincott Williams & Wilkins, 2000. Other modifications include a change in any halogen to any other halogen (for example, a F atom can be changed to a Cl atom, a Br atom or an I atom).

Thus, the present invention relates to compositions for treating patients that have cardiovascular diseases and other vascular related diseases using the above enumerated composition(s).

The present invention also allows for the addition of one or more adjuncts including but not limited to other lipid lowering drugs and associated drugs such as ezetimibe (ZETIA), niacin, pioglitazone (ACTOS)(if used for DM), carvedilol(COREG) (if used for patients that are HTN (hypertensive) / CHF (congestive heart failure)/ post MI), and/or low dose fibrates.

Other components that can be added to the composition of the instant invention include for example, cholesterol transferase inhibitors, phytosterols and/orphytostanols, mevinolin (compactin), omega-3 fatty acids, and other agents. For these agents and additional disclosure relating to these and other agents that act as inhibitors please See Brown, M. S. and Goldstein, J. L. (1993) in The Pharmacological Basis of Therapeutics (8.sup.th Ed.) Gilman, A. G. et al. eds. McGraw-Hill/New York, pp. 874-896.

### Clinical Results

The protocol of the instant invention was developed for patients that are high risk for a life and/or limb threatening vascular event (MI, CVA, limb/part of limb loss, Aortic dissection/rupture, Renal infarction, etc.) such as DM type I and/or II, CAD, PVD, post MI, post CVA, Hypertriglyceridemia, Hypercholesterolemia, AAA, etc. The treatment of the patients with the composition of the present invention led to improved symptoms and improved health of those patients, including but not limited to the resolution/reduction of angina, DOE (dyspnea on exertion), PVD signs/symptoms, Osteoarthritis, Erectile Dysfunction, normalization of liver function tests (LFTs) / Stress tests, improved feelings of wellbeing / no events (MI /CVA /threatened limb) in approximately 11 to 12 months in compliant patients.

Generally, very high risk vascular patients have LDL cholesterol levels that are 200 mg/dL or more (see Table 1 above) and no single statin when combined with any adjuncts, adjuvant, or other metabolite can bring them to a desired level. However, a small dose of, for example, simvastatin (20mg) plus pravastatin (40 mg) can lower a patient's LDL cholesterol level to goals of therapy (resolution/reduction of signs/symptoms/risks of vascular diseases). In one instance, a patient's LDL cholesterol level was reduced to virtually zero (one patient had a calculated level of negative 2 mg/dL in 2 months) without any other complaints.

When at least one or more lipophilic statins was combined with one or more hydrophilic statins results were obtained that were at least additive in LDL cholesterol lowering and in many cases were synergistic (greater than additive) to lower LDL cholesterol levels.

For example, in an instance, a patient taking one dose the first day lowered his LDL from 201 (measured 3 months ago but no medication taken for cholesterol lowering and no change in current meds/weight/diet) to 118 in less than a one day (the patient was instructed to hold statins until baseline cholesterol panel could be rechecked the next morning but the patient forgot and took the medications that evening) period by taking a simvastatin /pravastatin composition. The HDL level remained largely unchanged, triglycerides were up about 20 points (this is believed to be a temporary effect). From other patients' results, it is believed that the lipid profile will improve even more in one month. When adding adjuncts such as low dose Niacin (500 mg) and/or ezetimibe 10 mg, etc., the LDL lowering effects are also amplified. It is generally acknowledged that a goal for a DM/CAD patient is to have a LDL cholesterol level less than 70 mg/dL (National Cholesterol Education Program Interim Report Guidelines). When one attains an LDL cholesterol level approximately 70 mg/dL, plaques stabilize (do not continue to reduce lumen diameter) per IVUS (Intravascular Ultrasound) studies but still partially occlude (catheter design of IVUS does not allow measurement of completely occluded vessels). At a LDL cholesterol level of 30 to 40 mg/dL and HDL in normal range, plaques regress rapidly (this is a subjective standard, which can be seen both clinically and in imaging studies).

Patients with adverse reactions (allergy / laboratory abnormality / drug to (drug / herbal / food) interaction, etc.) to one statin did not mean that they would react adversely to all statins. A tiny dose of statin still has a significant benefit if tolerable. Very small doses of rosuvastatin (2.5mg/week to 5 mg/day) combined with a lipophilic statin combination such as a simvastatin /atorvastatin/ezetimibe combination has led to remarkable patient improvements (as seen in the laboratory, clinically and subjectively). About half of the very high risk vascular patients normally would have had another MI/CVA/ Vascular event this year based on patient history and statistical likelihood of patients with their initial levels of LDL cholesterol. However, those patients that mostly complied with dual statin therapy failed to have even one event. Moreover, those patients that are compliant appear to have Liver Function Tests that are superior to the patient's norm / initial level.

In other instances, patients with "sleepy feet" were now "waking up" and had pedal pulses that were now palpable (after as little as one month therapy). Multiple patients also report significant increases in walking distance (the patients could walk 20-200 ft initially, but after treatment could walk ¼-3 miles now).

### Regimen:

Generally, a patient should start with a low dose of one statin, the dose should then be titrated up to half a maximal dose (titrate means to start a patient at the lowest dose and increase the dose monthly based on having a patient that is less than 3X normal levels for the LFTs, the LDL cholesterol level is not to goal, and there is no positive change in signs, symptoms or studies to evaluate vascular function per disease state). At that point, the patient should be evaluated to see if the LDL cholesterol level is near the goal. If the patient is near the goal, adjuncts should be given to see if the LDL cholesterol level can be further lowered. If the patient is not near the LDL cholesterol goal after titration up to half a maximal dose, the dose of the initial statin should be reduced and a low dose second statin from the other group should be added to the regimen (i.e., if the initial statin is a hydrophilic statin, the second statin should be a lipophilic statin; and vice versa). The second statin level should be titrated up to a half maximal dose if upon taking the statin from the other group (i.e., the second statin), the LDL cholesterol level goal is still not attained. If one is near or at the LDL cholesterol level goal before or after titration of the second statin, the adjuncts can be added to further reduce the LDL cholesterol level. Not only has a synergistic relationship been found by using a statin from a lipophilic group and a statin from a hydrophilic group, but it has also been found that using adjuncts with this composition leads to enhanced effects for those adjuncts. For example, using a dose that includes either of ezetimibe 10 mg a day (20-25%) and/or niacin 500 mg a day (HDL up at least 10%) with the composition of statins, leads to effects that are not seen in the absence of the combination approach (i.e., having a composition comprising a lipophilic group and a hydrophilic group statin).

If significant Hypertriglyceridemia is present in a patient (this generally means that the triglyceride level (TRIG) is greater than about 400 mg/dL), a patient should first be treated to lower triglyceride levels so that one can obtain accurate levels of HDL and LDL. Once the triglyceride levels are below about 200 (and if said patient is in a high risk category), a statin therapy as indicated above should be initiated to lower the LDL cholesterol level to the 30 to 40 range (physiologic and usual level of benefits noted).

If the HDL cholesterol level in a non-alcoholic patient is >59, the benefits noted above can happen with a LDL cholesterol level in the 60's. If the TRIG level is greater than 1000, the patient should be started with bile binders and a statin at the same time. Based on the patient's history, current medicines being taken by the patient and any possible statin reaction and/or allergy history will determine with which statin to start. If, for example, the patient had a reaction to a lipophilic statin (such as atorvastatin or simvastatin, for example) that is not an indication that they will react adversely to a hydrophilic statin (such as pravastatin and/or rosuvastatin, for example) and vice versa. Most adverse reactions to statins tend to occur at the higher/highest dose. Thus, the regimen of the present invention that tends to use much lower doses will likely be more effective. Thus, in one embodiment, a regimen to employ is to titrate the statin of choice to a maximum not to exceed one half of the maximal manufacturer's recommended dose (if the patient has a heavy medicine load, pravastatin is a preferred statin to initiate treatment based on a low liver processing load and the lowest side effect incidence). If the LDL cholesterol level is above 200, rosuvastatin is usually the best statin to use for large initial reduction. If the patient is near the goal LDL cholesterol level (within 30 points), adjuncts such as ezetimibe, niacin, pioglitazone (if DM), carvedilol (if HTN / CHF / post MI), or low dose fibrates may be added and/or substituted appropriately. If not close to the LDL cholesterol level goal (i.e., greater than 30 points away from LDL cholesterol goal), the patient should be medicated with a second statin. If one sees an LDL cholesterol level reduction of less than 40-50% with the first statin tried, the first statin should be changed for another and titration performed as described above. The second statin should be initiated from the other/opposite class (*i.e.*, if a first statin is hydrophilic than the second statin should be lipophilic and vice versa) for a maximal lowering effect.

A hydrophilic statin plus a lipophilic statin shows a synergistic effect that is at least additive and in many patients greater than this. In one patient, a maximal reduction of an LDL cholesterol level was found to be 101 % with said patient registering a LDL cholesterol level that was -2.

If upon initial treatment of the patient with a statin at half maximum recommended dose produces an LDL cholesterol level that is near the LDL predetermined goal (i.e., only 30 to 40 points away from the goal), the first statin dose should be halved upon starting the second statin, which should be given at the lowest manufactured dose to prevent negative LDL cholesterol levels (the patient showing the 2 LDL cholesterol level referenced above had no complaints and other indicative laboratory results were within normal limits).

In the situation where a patient is being titrated with the statins to half maximal doses and the patient is still not near the LDL cholesterol goal, the patient should be questioned and/or assessed for non-compliance with the regimen. The patient should also be assessed for other causes of cholesterol elevation (assuming DM/Hypothyroidism and the like are controlled).

In an embodiment, if adding a second statin at half maximal dose results in LDL cholesterol levels that are not near the goal, the second statin should be changed if the first statin worked well (i.e., approximately 50% reduction or more). If changing the second statin does not work well, the first statin should be changed. The composition comprising the two statins amplify the effectiveness of the above noted adjuncts, therefore those adjuncts can be used at low doses as well. For example, using niacin (B-3) at a dose of 500 mg to 750 mg a day for an adult gives a 10% or more boost to the HDL levels. The blend in most people will lower the HDL level on the order of about 10-20%, but this is most likely the atherogenic small dense LDL, as it is known that vascular disease in all beds tend to reverse rapidly (i.e., the average time to noted subjective improvement is about 1-2 months). The Lpa and Apo-B levels were not assessed for these patients.

Also some patients have a mild increase in triglycerides, but a half dose of bile binders (if their triglyceride level is above 200) will easily return those patients to their normal levels. Generally, it is prudent and recommended to use statins more than one hour before or about 4-6 hours after a bile binder (for example, in the embodiments of the invention colesevelam is mainly used and rarely cholestyramine (less tolerated) is used).

In an embodiment, the present invention relates to a composition comprising one or more lipophilic statins and one or more hydrophilic statins. The composition optionally further comprising one or more adjuncts.

In an embodiment, the one or more lipophilic statins is selected from the group consisting of atorvastatin, simvastatin, and lovastatin. In an embodiment, the one or more hydrophilic statins is selected from the group consisting of pravastatin, fluvastatin and rosuvastatin. In another embodiment, the one or more lipophilic statins is selected from the group consisting of atorvastatin, simvastatin, and lovastatin and the one or more hydrophilic statins is selected from the group consisting of pravastatin, fluvastatin and rosuvastatin. This composition may further comprise one or more adjuncts.

In another embodiment, the present invention provides a method of treating a patient with a high risk of cardiovascular disease and/or other vascular related symptoms and/or one or more cardiovascular diseases comprising administering to said patient a pharmaceutically acceptable dose of a composition comprising one or more lipophilic statins and one or more hydrophilic statins. The method uses a composition that optionally further comprises one or more adjuncts.

In one form the method uses one or more lipophilic statins that are selected from the group consisting of atorvastatin, simvastatin, and lovastatin. In one form the method uses one or more hydrophilic statins that are selected from the group consisting of pravastatin, fluvastatin and rosuvastatin. In another form the method uses one or more lipophilic statins that are selected from the group consisting of atorvastatin, simvastatin, and lovastatin and the one or more hydrophilic statins are selected from the group consisting of pravastatin, fluvastatin and rosuvastatin. The method using this composition may further comprise one or more adjuncts.

In one form the method reduces and/or reverses the high risk of cardiovascular disease and/or other vascular related symptoms and/or one or more cardiovascular diseases.

In another form the invention provides a method of treating a patient with a high risk of cardiovascular disease and/or other vascular related symptoms and/or one or more cardiovascular diseases comprising:
initiating treatment of said patient by administering to said patient a low dose of a first statin;
titrating said first statin up to a half maximal dose;
and evaluating a LDL cholesterol level to ascertain if said patient is near a predetermined goal.

Generally, the predetermined goal is between about 40% to about 50% of the initial starting LDL cholesterol level of a patient prior to treatment. By "near", it is meant that the patient is about at or below 30 mg/dL from the predetermined goal. In an exemplary embodiment, if an initial starting LDL cholesterol level for a patient prior to treatment is 250 mg/dL, a predetermined goal would be 125 mg/dL. If the patient is at or below about 155 mg/dL, they are "near" the predetermined goal. In contrast, a patient that is "far from" a predetermined goal is above about 30 mg/dL from said predetermined goal. In the exemplary embodiment described above, the patient that is "far from" the predetermined goal will have an LDL cholesterol level that is above about 155 mg/dL. It should be understood that the predetermined goal will be dependent upon a number of factors such as the relative levels of LDL cholesterol as well as the patient history. In an embodiment, the predetermined goal is about or less than 100 mg/dL, or alternatively, the predetermined goal is about or less than 125 mg/dL, or alternatively, the predetermined goal is about or less than 130 mg/dL.

In a form if said patient is near said predetermined goal after evaluating the LDL cholesterol level, then the method optionally involves adding one or more adjuncts to further reduce the LDL cholesterol level.

If adding one or more adjuncts fails to further reduce or only moderately reduces the LDL cholesterol level, the method optionally allows lowering the dose of the first statin and administering a low dose of a second statin.

The first and the second statin are from different groups. That is, if the first statin is a hydrophilic statin, then the second statin is a lipophilic statin. Alternatively, if the first statin is a lipophilic statin then the second statin is a hydrophilic statin.

If the patient is far from the predetermined goal after evaluating the LDL cholesterol level, the method optionally further allows reducing the dose of the first statin; and administering a low dose of a second statin. If the patient is far from the predetermined goal after evaluating the LDL cholesterol level, the method uses a first statin that is a hydrophilic statin, and a second statin that is a lipophilic statin. Alternatively, if the first statin is a lipophilic statin, then the second statin is a hydrophilic statin.

Optionally if administering a low dose of a second statin fails to further lower or only moderately lowers the LDL cholesterol level, then one should titrate the second statin to ascertain if said LDL cholesterol level further decreases.

If titrating the second statin fails to further decrease said LDL cholesterol level, the method allows optionally replacing said second statin with a third statin at a low dose.

## Claims

1. A composition comprising one or more lipophilic statins and one or more hydrophilic statins for use in treating a patient with a high risk of cardiovascular disease and/or other vascular related symptoms and/or one or more cardiovascular diseases in order to reach a pre-determined goal wherein the composition is to be administered using the following titration method to determine the dose of a first and second statin:
i) titrating a low dose of a first statin up to a half maximal dose;
ii) evaluating a LDL cholesterol level to ascertain if said patient is near a predetermined goal;
iii) if said patient is far from said predetermined goal after evaluating the LDL cholesterol level, reducing the dose of the first statin and adding a low dose of a second statin to the regimen
wherein if the first statin is a hydrophilic statin, then the second statin is a lipophilic statin; wherein if the first statin is a lipophilic statin then the second statin is a hydrophilic statin.

2. A composition for use according to claim 1 comprising the further step of iv) evaluating a LDL cholesterol level to ascertain if said patient is near a predetermined goal;
v) if said low dose of a second statin fails to further lower or only moderately lowers said LDL cholesterol level, titrating said second statin to ascertain if said LDL cholesterol level further decreases.

3. A composition for use according to claim 1 and 2 comprising the further step of
vi) evaluating a LDL cholesterol level to ascertain if said patient is near a predetermined goal;
vii) if titrating said second statin fails to further decrease said LDL cholesterol level, replacing said second statin with a third statin at a low dose.

4. A composition for use according to any of claims 1 to 3 comprising the further intermediate step of
iia) adding one or more adjuncts to further reduce the LDL cholesterol level.

5. A composition for use according to any of claims 1 to 4 comprising the further intermediate step of
iiia) adding one or more adjuncts to further reduce the LDL cholesterol level.

6. A composition for use according to claim 1, wherein said predetermined goal is about or less than 100 mg/dL.

7. A composition for use according to claim 1, wherein the one or more lipophilic statins is selected from the group consisting of atorvastatin, simvastatin, and lovastatin.

8. A composition for use according to claim 1, wherein the one or more hydrophilic statins is selected from the group consisting of pravastatin, fluvastatin and rosuvastatin.

## Patentansprüche

1. Zusammensetzung, die ein oder mehr lipophile Statine und ein oder mehr hydrophile Statine umfasst, zur Verwendung bei der Behandlung eines Patienten mit einem hohen Risiko einer kardiovaskulären Erkrankung und/oder anderen gefäßbezogenen Symptomen und/oder einem oder mehr kardiovaskulären Erkrankungen, um einen vorbestimmten Zielwert zu erreichen, wobei die Zusammensetzung unter Verwendung des folgenden Titrationsverfahrens zur Bestimmung der Dosis eines ersten und zweiten Statins zu verabreichen ist:
i) Auftitrieren einer niedrigen Dosis eines ersten Statins auf eine halbe Höchstdosis;
ii) Auswerten eines LDL-Cholesterolspiegels, um festzustellen, ob der Patient in der Nähe eines vorbestimmten Zielwerts liegt;
iii) wenn der Patient nach dem Auswerten des LDL-Cholesterolspiegels weit von dem vorbestimmten Zielwert entfernt ist, Reduzieren der Dosis des ersten Statins und Hinzunehmen einer niedrigen Dosis eines zweiten Statins zu dem Therapieschema,
wobei in dem Fall, dass das erste Statin ein hydrophiles Statin ist, das zweite Statin ein lipophiles Statin ist;
wobei in dem Fall, dass das erste Statin ein lipophiles Statin ist, das zweite Statin ein hydrophiles Statin ist.

2. Zusammensetzung zur Verwendung nach Anspruch 1, die den weiteren Schritt umfasst
iv) des Auswertens eines LDL-Cholesterolspiegels, um festzustellen, ob der Patient in der Nähe eines vorbestimmten Zielwerts liegt;
v) in dem Fall, dass die niedrige Dosis eines zweiten Statins nicht in der Lage ist, den LDL-Cholesterolspiegel weiter zu senken, oder ihn nur mäßig senkt, des Titrierens des zweiten Statins, um festzustellen, ob der LDL-Cholesterolspiegel sich weiter vermindert.

3. Zusammensetzung zur Verwendung nach Anspruch 1 und 2, die den weiteren Schritt umfasst
vi) des Auswertens eines LDL-Cholesterolspiegels, um festzustellen, ob der Patient in der Nähe eines vorbestimmten Zielwerts liegt;
vii) in dem Fall, dass das Titrieren des zweiten Statins nicht in der Lage ist, den LDL-Cholesterolspiegel weiter zu vermindern, des Ersetzens des zweiten Statins durch ein drittes Statin in einer niedrigen Dosis.

4. Zusammensetzung zur Verwendung nach einem der Ansprüche 1 bis 3, die den weiteren Zwischenschritt umfasst
iia) des Hinzunehmens von ein oder mehr Adjuvantien, um den LDL-Cholesterolspiegel weiter zu reduzieren.

5. Zusammensetzung zur Verwendung nach einem der Ansprüche 1 bis 4, die den weiteren Zwischenschritt umfasst
iiia) des Hinzunehmens von ein oder mehr Adjuvantien, um den LDL-Cholesterolspiegel weiter zu reduzieren.

6. Zusammensetzung zur Verwendung nach Anspruch 1, wobei der vorbestimmte Zielwert etwa oder weniger als 100 mg/dl ist.

7. Zusammensetzung zur Verwendung nach Anspruch 1, wobei das eine oder mehr lipophile Statin aus der Gruppe ausgewählt ist, die Atorvastatin, Simvastatin und Lovastatin umfasst.

8. Zusammensetzung zur Verwendung nach Anspruch 1, wobei das eine oder mehr hydrophile Statin aus der Gruppe ausgewählt ist, die Pravastatin, Fluvastatin und Rosuvastatin umfasst.

## Revendications

1. Composition comprenant une ou plusieurs statines lipophiles et une ou plusieurs statines hydrophiles, utilisable pour traiter un patient présentant un risque élevé de maladie cardiovasculaire et/ou d'autres symptômes d'ordre vasculaire et/ou une ou plusieurs maladies cardiovasculaires afin d'atteindre un objectif prédéterminé, laquelle composition doit être administrée en utilisant le procédé de titrage suivant pour déterminer la dose d'une première et d'une deuxième statine :
i) titrage d'une faible dose d'une première statine jusqu'à la moitié de la dose maximale ;
ii) évaluation d'un taux de cholestérol LDL pour déterminer si ledit patient est proche d'un objectif prédéterminé ;
iii) si ledit patient est loin dudit objectif prédéterminé après évaluation du taux de cholestérol LDL, réduction de la dose de la première statine et ajout d'une faible dose d'une deuxième statine dans le régime posologique
dans laquelle, si la première statine est une statine hydrophile, alors la deuxième statine est une statine lipophile ;
dans laquelle, si la première statine est une statine lipophile, alors la deuxième statine est une statine hydrophile.

2. Composition utilisable selon la revendication 1, comprenant l'étape supplémentaire consistant à :
iv) évaluer un taux de cholestérol LDL pour déterminer si ledit patient est proche d'un objectif prédéterminé ;
v) si ladite faible dose d'une deuxième statine ne parvient pas à réduire davantage le taux de cholestérol LDL ou réduit seulement modérément ledit taux de cholestérol LDL, titrer ladite deuxième statine pour déterminer si ledit taux de cholestérol LDL diminue davantage.

3. Composition utilisable selon la revendication 1 ou 2, comprenant l'étape supplémentaire consistant à :
vi) évaluer un taux de cholestérol LDL pour déterminer si ledit patient est proche d'un objectif prédéterminé ;
vii) si le titrage de ladite deuxième statine ne parvient pas à réduire davantage le taux de cholestérol LDL, remplacer ladite deuxième statine par une troisième statine à une faible dose.

4. Composition utilisable selon l'une quelconque des revendications 1 à 3, comprenant l'étape intermédiaire supplémentaire consistant à :
iia) ajouter un ou plusieurs additifs pour réduire davantage le taux de cholestérol LDL.

5. Composition utilisable selon l'une quelconque des revendications 1 à 4, comprenant l'étape intermédiaire supplémentaire consistant à :
iiia) ajouter un ou plusieurs additifs pour réduire davantage le taux de cholestérol LDL.

6. Composition utilisable selon la revendication 1, dans laquelle ledit objectif prédéterminé est environ égal ou inférieur à 100 mg/dl.

7. Composition utilisable selon la revendication 1, dans laquelle la ou les statine(s) lipophile(s) est/sont choisie(s) dans le groupe constitué par l'atorvastatine, la simvastatine et la lovastatine.

8. Composition utilisable selon la revendication 1, dans laquelle la ou les statine(s) hydrophile(s) est/sont choisie(s) dans le groupe constitué par la pravastatine, la fluvastatine et la rosuvastatine.
